# EUROPÄISCHE PATENTSCHRIFT

(11) **EP 1 968 900 B1**
(45) Veröffentlichungstag und Bekanntmachung des Hinweises auf die Patenterteilung: **08.06.2011**
(21) Anmeldenummer: 06829809.0
(22) Anmeldetag: 21.12.2006
(51) Int. Cl.: C02F 1/44, C02F 3/12, C02F 3/28, C02F 3/34, C02F 1/28, C02F 1/42, C02F 101/10, C02F 101/16

(54) **ANAEROBE REINIGUNG VON ABWASSER**
ANAEROBIC PURIFICATION OF WASTEWATER
EPURATION ANAEROBIE D'EAUX USEES

(30) Priorität: 23.12.2005 DE 102005063228
(43) Veröffentlichungstag der Anmeldung: 17.09.2008
(73) Patentinhaber: Fraunhofer-Gesellschaft zur Förderung der angewandten Forschung e.V., 80686 München (DE)
(72) Erfinder: STERNAD, Werner, 70197 Stuttgart (DE); SPORK, Christian, 86154 Augsburg (DE); MOHR, Marius, 70192 Stuttgart (DE); TRÖSCH, Walter, 70329 Stuttgart (DE); TRICK, Iris, 75305 Neuenbürg (DE); KRISCHKE, Wolfgang, 72138 Kirchentellinsfurt (DE)
(74) Vertreter: Schwahn, Hartmut
(86) Internationale Anmeldenummer: PCT/EP2006/012375
(87) Internationale Veröffentlichungsnummer: WO 2007/076953

(56) Entgegenhaltungen:
- WO-A-03/039712
- DE-C1- 19 580 599
- JP-A- 10 118 687
- US-A1- 2004 007 533
- LETTINGA G ET AL: "Challenge of psychrophilic anaerobic wastewater treatment" TRENDS IN BIOTECHNOLOGY, ELSEVIER PUBLICATIONS, CAMBRIDGE, GB, Bd. 19, Nr. 9, 1. September 2001 (2001-09-01), Seiten 363-370, XP004300204 ISSN: 0167-7799
- DAGUE R R ET AL: "ANAEROBIC SEQUENCING BATCH REACTOR TREATMENT OF DILUTE WASTEWATER AT PSYCHROPHILIC TEMPERATURES" WATER ENVIRONMENT RESEARCH, WATER ENVIRONMENT FEDERATION, ALEXANDRIA, VA, US, Bd. 70, Nr. 2, 1. März 1998 (1998-03-01), Seiten 155-160, XP000769909 ISSN: 1061-4303
- REBAC S ET AL: "HIGH-RATE ANAEROBIC TREATMENT OF MALTING WASTE WATER IN A PILOT-SCALE EGSB SYSTEM UNDER PSYCHROPHILIC CONDITIONS" JOURNAL OF CHEMICAL TECHNOLOGY AND BIOTECHNOLOGY, WILEY & SONS, CHICHESTER, GB, Bd. 68, Nr. 2, Februar 1997 (1997-02), Seiten 135-146, XP000690919 ISSN: 0268-2575
- BAILEY A D ET AL: "THE ENHANCEMENT OF UPFLOW ANAEROBIC SLUDGE BED REACTOR PERFORMANCE USING CROSSFLOW MICROFILTRATION" WATER RESEARCH, ELSEVIER, AMSTERDAM, NL, Bd. 28, Nr. 2, 1. Februar 1994 (1994-02-01), Seiten 291-295, XP000415299 ISSN: 0043-1354
- DATABASE WPI Section Ch, Week 200511 Derwent Publications Ltd., London, GB; Class D15, AN 2005-101839 XP002427418 & WO 2005/005328 A (EBARA CORP) 20. Januar 2005 (2005-01-20)

## Beschreibung

Die vorliegende Erfindung betrifft Verfahren zur Reinigung von Abwasser, beispielsweise kommunale Abwässer, mittels anaerober biologischer Reinigung, Verfahren zur Gewinnung von Biogas aus Abwasser sowie Verwendung von Vorrichtungen, die zur anaeroben biologischen Reinigung von Abwasser geeignet sind.

### Stand der Technik

Vorrichtungen und Verfahren zur Reinigung von Abwasser, vor allem von kommunalen Abwässern, sind bekannt. Beispiele dafür sind die sogenannten Kläranlagen beziehungsweise Abwasserreinigungsanlagen. Diese dienen primär der Reinigung kommunaler Abwässer, die von der öffentlichen Kanalisation gesammelt und zur Kläranlage transportiert werden. Das Ziel der Abwasserreinigung ist die Entfernung unerwünschter Bestandteile aus dem Abwasser, sodass ein gereinigtes Abwasser erhalten wird, das beispielsweise in Flüsse und Gewässer (so genannte Vorfluter) freigesetzt werden kann, ohne dass Schäden für die Umwelt und die Bevölkerung durch chemische oder mikrobiologische Belastung auftreten.

Zur Entfernung der unerwünschten Bestandteile werden, meist in Kombination, mechanische (physikalische), biologische und chemische Verfahren eingesetzt. Moderne Kläranlagen sind dementsprechend dreistufig, wobei mindestens eine physikalische Reinigungsstufe, mindestens eine biologische Reinigungsstufe und mindestens eine chemische Reinigungsstufe hintereinander geschaltet sind. Biologische Verfahren dienen vor allem dem Abbau von organischen Verbindungen in organisch hoch belasteten Abwässern. Dies erfolgt bekanntermaßen durch aeroben Abbau der organischen Verbindungen zu anorganischen Endprodukten wie Kohlenstoffdioxid, Nitrat, Sulfat, Phosphat, etc. Dazu wird Biomasse in Form von Belebtschlämmen in sogenannten Belebungsbecken beziehungsweise als Bakterienrasen zum Beispiel in Form sogenannter Tropfkörper eingesetzt. Die zu entfernenden organischen Verbindungen werden dabei nicht nur durch katabolische Vorgänge energetisch verwertet und "veratmet", d.h. durch Umsetzung mit Luftsauerstoff abgebaut, sondern dienen in anabolischen Vorgängen dem Wachstum der Biomasse. Die sich anreichernde Biomasse muss regelmäßig aus der biologischen Reinigungsstufe entfernt und in einem Faulturm einer Nachbehandlung, zum Beispiel einer Vergärung unterzogen werden, wobei Biogas freigesetzt und trotzdem noch erhebliche Mengen Schlammabfall überbleiben. Dieser Schlammabfall kann teilweise als Dünger verwendet werden. Zum Großteil wird das Schlammaufkommen jedoch kostenträchtig in Verbrennungsanlagen beseitigt.

Bekannt ist auch eine anaerobe Abwasserreinigung. Diese dient ebenfalls der Entfernung schädlicher oder störender organischer Kohlenstoffvarbindungen im Abwasser durch mikrobiologische Abbauprozesse, die jedoch in Abwesenheit von Sauerstoff ablaufen. Dabei gewinnen die anaeroben Mikroorganismen die für ihren Stoffwechsel erforderliche Energie aus der Umsetzung der organischen Kohlenstoffverbindungen und setzen diese zu organischen Säuren, anderen Kohlenwasserstoffen, zu Kohlendioxid und Methan um. Die Abwasserreinigung findet dabei in luftdicht verschlossenen Reaktoren statt. Um die erforderliche Reinigungsaktivität ― gemessen durch den Abbau der Abwasserbelastung an oxidierbaren Verbindungen ― zu erreichen, ist zumeist eine Beheizung des Abwassers zum Erreichen des für das Wachstum der Bakterien erforderlichen Temperaturoptimums nötig.

Allgemein unterteilt man Anaerobverfahren in Durchflusssysteme ohne Immobilisierung der Biomasse, worin die Biomasse im Wesentlichen in Suspension vorliegt, und in Durchflusssysteme mit Immobilisierung, worin die Biomasse im Wesentlichen an innere Strukturen des Reaktors immobilisiert vorliegt und von dem zu reinigenden Abwasser umströmt wird.

Bekannt ist der sogenannte "Emscherbrunnen", ein von Immhoff 1906 patentierter zweistufiger Klärturm der im unten liegenden Faulraum eine anaerobe Faulung ermöglicht. Die durchschnittliche Ausfaulzeit des Schlammes beträt dabei, abhängig von der Abwassertemperatur, etwa 3 Monate. Diese Anlage wird in der Regel zur Vorklärung, nicht aber zur Hauptklärung und Reinigung des Abwassers eingesetzt.

Beispielsweise entsteht bei der Verarbeitung von Zuckerrüben in Zuckerfabriken hochbelastetes Abwasser, welches durch bekannte anaerobe Abwasserreinigungsanlagen gereinigt wird. Dabei werden die Abwässer zweistufig abgebaut: In einer ersten Stufe, der sogenannten Versäuerung, werden die im Abwasser enthaltenen hochmolekularen Zuckerstoffe und andere organische Verbindungen durch Mikroorganismen zu organischen Säuren umgebaut. Diese Säuren sind das abbaubare Substrat für die Bakterien in der zweiten Stufe, der sogenannten methanogenen Phase. Die methanogene Phase ist dabei die mikrobielle Phase, die das Abwasser reinigt, indem CSB (gelöste oxidierbare organische Substanz) zu gasförmigen Produkten CH₄ und CO₂ umgewandelt werden, die als solche das Wasser kostenlos und freiwillig verlassen. Die zweite Phase kann unter anderem in Form eines Fließbettreaktors ausgebildet sein. Dazu wird das Abwasser in der Versäuerungsstufe über einen Wärmetauscher auf zirka 38 °C angewärmt.

Neben Fließbettreaktoren werden zur bekannten anaeroben Abwasserreinigung auch Faultürme, anaerobe Belebungsbecken, UASB-Reaktoren oder Festbettreaktoren eingesetzt.

Die bei bekannten anaeroben Reinigungsverfahren erreichten Restkonzentrationen organischer Verunreinigungen erlauben keine direkte Einleitung in den Vorfluter. Es sind daher weitere aerobe biologische und/oder chemische Reinigungsstufen erforderlich.

In bekannten Anaerobreaktoren sind aktive Schlammkonzentrationen bis maximal 20 g/l erreichbar. Bekannte Verfahren besitzen eine Schlammbelastung von etwa 0,2 bis 0,3 g CSB (chemischer Sauerstoffbedarf, bei Oxidation durch Kaliumdichromat) pro Gramm Trockensubstanz des Schlammes pro Tag. Für den Abbau von 1 g/l CSB pro Tag sind bekanntermaßen 3 bis 5 g Trockensubstanz in Form von anaerober Biomasse im Reaktor vorzuhalten.

Weiter sind bekannte anaerobe Reinigungsverfahren sensibel im Hinblick auf die Zutaufbeschaffenheit und die Einhaltung von Betriebsbedingungen, insbesondere der Zulauftemperatur. Bekannte Verfahren sind empfindlich gegenüber Temperaturveränderungen des eingeleiteten Abwassers. Die Einleittemperaturen von kommunalen Abwässern betragen in der Regel zwischen 4 und 30 °C. Gerade bei niedrigen Temperaturen, das heißt weniger als 20°C, können bekannte anaerobe Verfahren ohne weitere Maßnahmen der Temperierung der Abwässer, diese nicht ausreichend reinigen.

Bei Durchflusssystemen ohne Immobilisierung führen hydraulische Verweilzeiten, die die momentan exisiterende Wachstumsgeschwindigkeit der Methanbakterien bei gegebenen Betriebsbedingungen überschreiten, zum Auswaschen dieser Biokatalysatoren und zum Zusammenbrechen des Reinigungsprozesses. Die momentan existierende Wachstumsgeschwindigkeit kann unter anderem durch Temperatur, pH-Wert sowie Ammoniak-, H₂S- und Salzkonzentration verändert werden. Da die acidogene Phase weniger empfindlich auf Wachstumseinflüsse reagiert als die methanogene Phase, neigen insbesondere Reaktoren im psychrophilen Temperaturbereich, zum Beispiel bei sehr kleinen Wachstumsgeschwindigkeiten (T*aktuell* << T*optimal*) von Methanbakterien, zur Versäuerung.

Werden Reaktoren verwendet, die aufgrund sogenannter "wash out"-Problematik Bakterien immobilisieren (Durchflusssysteme mit Immobilisierung), erreichen diese aufgrund von Diffusionslimitierung in den Immobilisierungsschichten keine Reinigungsleistung, die für Einleitung in Vorfluter ausreicht.

Aus Lettinga et al. (Challenge of Psychrophilic Anaerobic Wastewater Treatment. TRENDS in Biotechnology, 2001, 19(9):363-369) ist die Abwasserreinigung und Methanisierung unter psychrophilen Bedingungen in einem "sludge bed"-Reaktor bekannt. Aus Dague et al. (Anearobic Sequencing Batch Reactor Treatment of Dilute Wastewater at Psychrophilic Temperatures. Water Environment Research, 1998, 70(2):155-160) ist die methanisierende Abwasserreinigung unter psychrophilen Bedingungen in einem "Sequencing Batch"-Bioreaktor bekannt. Aus Rebac et al., (High-rate Anaerobic Treatment of Malting Waste Water in a Pilot-Scale EGSB System Under Psychorophilic Conditions. J. Chem. Tech. Biotechnol. 1997, 68:135-146) ist die methanisierende Reinigung von Brauereiabwässem unter psychrophilen Bedingungen unter Verwendung eines "Expanded Granular Sludge Bed"-Reaktor bekannt; dabei wird die anaerobe Abwasserreinigung als Option für gering belastete Abwässer beschrieben.

### Aufgabenstellung

Das der vorliegenden Erfindung zugrunde liegende technische Problem besteht vor allem darin, ein verbessertes Verfahren zur anaeroben biologischen Reinigung von Abwasser bereitzustellen, welches eine geringe Temperaturempfindlichkeit aufweist und eine hohe Reinigungseffektivität beim Abbau organischer Verunreinigungen zeigt.

Das technische Problem wird gelöst durch ein Verfahren zur Reinigung von Abwasser gemäß Anspruch 1, worin mindestens ein Schritt einer anaeroben biologischen Reinigung, das heißt Reinigungsstufe, durchgeführt wird. Das erfindungsgemäße Verfahren im Wesentlichen ist dadurch gekennzeichnet, dass in der mindestens einen anaeroben biologischen Reinigungsstufe Biomasse eingesetzt wird, die ausgewählt ist aus psychrophilen Mikroorganismen. Vorzugsweise weisen die psychrophilen Mikroorganismen ein Temperaturoptimum auf, das bei einer Temperatur von weniger als 35 °C, bevorzugt weniger als 25 C, besonders bevorzugt weniger als 20 °C, liegt. Die Biomasse liegt erfindungsgemäß in einem sogenannten Reaktor oder Bioreaktor vor, und zwar suspendiert, bei Volldurchmischung des Reaktors. Erfindungsgemäß wird die Biomasse durch Mikrofiltration aufkonzentriert und in die anaerobe biologische Reinigung zurückgeführt. Erfindungsgemäß beträgt die Biomassekonzentration im Bioreaktor 25 g/l oder mehr.

Die Erfindung sieht also vor, mindestens eine anaerobe biologische Reinigungsstufe mittels psychrophiler Mikroorganismen durchzuführen. Der erfindungsgemäße Einsatz der psychrophilen Mikroorganismen erlaubt die nahezu vollständige Entfernung organischer Verunreinigungen auf CSB-Werte von 75 mg/l und weniger. Typischerweise erreicht eine erfindungsgemäß betriebene zweistufige Anlage (zwei Reaktoren hintereinandergeschaltet) Ablaufwerte von etwa 50 bis etwa 100 mg/l CSB bei einem Zulauf von etwa 3700 mg/l CSB. Dies bedeutet eine Elimination der anorganischen Substanzen von 97 bis 98,5 %. Die Zulauftemperatur beträgt dabei zwischen etwa 4 und etwa 35°C.

Mit dem erfindungsgemäßen Verfahren gelingt es also, allein durch anaerobe biologische Reinigung und ohne zusätzliche Maßnahmen wie Temperierung CSB-Werte zu erreichen, die europäischen Standards beziehungsweise die der deutschen Abwasserverordnung genügen.

Die Erfinder fanden überraschend, dass mittels psychrophiler Mikroorganismen eine effektive anaerobe biologische Reinigung von Abwasser durchgeführt werden kann. Dies gelingt vor allem, wenn die Biomassenkonzentration im Reaktor hoch gehalten wird. Die Biomasse weist erfindungsgemäß eine Konzentration von 25 g/l oder mehr, bevorzugt von 50 g/l oder mehr auf. Die obere Grenze liegt bei etwa 100 g/l. In einer bevorzugten Variante beträgt die Biomassenkonzentration von 25 g/l bis 100 g/l. Erfindungsgemäß wird ein volldurchmischter Reaktor eingesetzt. Erfindungsgemäß ist dabei am Ausgang des Reaktors eine geeignete Mikrofiltrationsvorrichtung, beispielsweise ein Rotationsscheibenfilter, vorgesehen, wodurch Rückhalten und gegebenenfalls Rückführen des aus dem Reaktor im hydraulischen Durchfluss entweichenden Schlamms, der Biomasse enthält, ermöglicht wird.

Wider Erwarten eignen sich psychrophile Mikroorganismen unter Anwendung geeigneter Verfahrensmaßnahmen zur effektiven Reinigung von Abwasser, wobei die bekannten Vorteile der anaeroben biologischen Reinigung wie kostengünstige Realisierung, Entfallen der energieaufwändigen Belüftung und vor allem die Vermeidung von ca. 90 % der unerwünschten Klärschlammproduktion zum Tragen kommen. Gleichzeitig kann mittels des erfindungsgemäßen Verfahrens auch auf einfache Weise, beispielsweise ohne zusätzlichen Faulturm, Biogas, das heißt im Wesentlichen ein Gemisch aus Methan und Kohlenstoffdioxid, das bei der anaeroben biologischen Reinigung entsteht, aus dem zu reinigenden Abwasser gewonnen werden.

Ohne an die Theorie gebunden zu sein, erlaubt die erfindungsgemäß konstant hohe Katalysatorkonzentration im Reaktor eine temperaturunabhängige Methanisierung, das heißt Stoffwechselaktivität der Biomasse, wodurch der Abbau der im Abwasser vorhandenen unerwünschten organischen Verbindungen gekennzeichnet ist. Bei kalten Zulauftemperaturen, vor allem unterhalb von 20°C, und/oder bei geringem Verschmutzungsgrad des Abwassers mit organischen Verunreinigungen fallen die in der biologischen Reinigungsstufe enthaltenen psychrophilen Mikroorganismen in einen sogenannten Erhaltungsstoffwechsel. Dabei findet die Abwasserreinigung in hoher Effektivität überraschenderweise bereits unter Bedingungen des Erhaltungsstoffwechsels (Maintenance-Bedingungen) statt.

Unter Maintenance-Bedingungen ist die Stoffwechselaktivität auch der Methanbakterien kleiner als unter Bedingungen, bei welchen die Bakterien wachsen können. Um dann trotzdem hohe Reinigungsleistungen erreichen zu können, muss die geringere Aktivität durch eine höhere Konzentration von Bakterien ausgeglichen werden. Deshalb wird bevorzugt der Prozess immer bei einer Bakterienkonzentration betrieben, die ausreichend ist, um die Biogasbildung auf einem Niveau zu halten, das zur Erreichung von CSB Einleitbedingungen ausreichend ist. Dies ist von erfindungsgemäß von 25 bis 100 g aktiver Biomasse erzielbar auch für Abwassertemperaturen, die sehr viel kleiner sind als 30°C.

Die erfindungsgemäße Wahl der Biomasse unter der Population psychrophiler Mikroorganismen erlaubt vor allem in Verbindung mit einer hohen Konzentration an Biomasse von erfindungsgemäß 25 g/l oder mehr in der biologischen Reinigungsstufe selbst bei tiefen Temperaturen von weniger als 20°C eine hoch effektive Abwasserreinigung. Dabei unterliegen die Mikroorganismen teilweise oder vollständig dem Erhaltungsstoffwechsel. Im Erhaltungsstoffwechsel wird vorteilhafterweise die unerwünschte Vermehrung der Biomasse vollständig unterdrückt, wodurch die nachteilige Schlammproduktion gänzlich vermieden wird.

Es zeigt sich, dass die erfindungsgemäße anaerobe biologische Reinigung hoch effektiv bereits in einem einstufigen Prozess als einstufige Methanisierung durchgeführt werden kann. Dabei wird vorteilhafterweise eine Eliminierung organischer Verbindungen von bis zu 95 % (gemessen anhand der CSB-Werte) erreicht. Wird das Verfahren in einem zweistufigen Prozess als zweistufige Methanisierung durchgeführt, werden bis zu 98,5 % der organischen Verbindungen (gemessen anhand der CSB-Werte) aus dem Abwasser eliminiert.

In einer bevorzugten Ausführungsform der Erfindung wird daher die anaerobe biologische Reinigung einstufig, vorzugsweise als einstufige Methanisierung, durchgeführt. In einer anderen bevorzugten Variante wird die anaerobe biologische Reinigung zweistufig, vorzugsweise als zweistufige Methanisierung, durchgeführt. Selbstverständlich können, je nach Anwendungsgebiet und Zweckmäßigkeit, auch mehr als zwei Stufen hintereinander geordnet, das heißt kaskadiert, werden. In der Regel reicht für die Anwendung bei kommunalen Abwässern eine zweistufige Methanisierung aus, um gereinigte Abwässer in einer Vorfluterqualität zu erhalten, die europäischen Standards und der deutschen Abwasserverordnung genügen.

Die Biomasse ist in dem zu reinigenden Abwasser suspendiert. Dies wird dadurch erreicht, dass die Biomasse in einem volldurchmischten Reaktor gehalten wird. Der aus dem Reaktor austretende Schlamm aus Biomasse und gereinigten beziehungsweise teilweise gereinigtem Abwasser wird wieder in den Reaktor zurückgegeben. Dies wird erreicht durch Abtrennen von Wasser aus dem austretenden Schlamm, wodurch der Schlamm aufkonzentriert wird. Anschließend wird der aufkonzentrierte Schlamm in die anaerobe biologische Reinigung zurückgeführt. Zur Rückgewinnung beziehungsweise Aufkonzentrierung des Schlamms wird mindestens eine Mikrofiltrationsvorrichtung verwendet. Vorzugsweise ist die Mikrofiltrationsvorrichtung dem Reaktor unmittelbar nachgeschaltet. Bevor zugt bilden Reaktor und Mikrofiltrationsvorrichtung dann eine integrierte Einheit. Es versteht sich, dass jede für diesen Zweck geeignete Mikrofiltrationsvorrichtung eingesetzt werden kann, um die Biomasse aus dem austretenden Schlamm zurückzugewinnen. Besonders bevorzugt wird als Mikrofiltrationsvorrichtung ein Rotationsscheibenfilter eingesetzt. Dieser erlaubt vorteilhafterweise den kontinuierlichen Betrieb bei hoher Effektivität. Ebenfalls bevorzugt sind Zentrifugen und/oder Dekantiersysteme. Es versteht sich ebenfalls, dass die Biomasserückhaltung auch im Reaktor selbst installiert werden kann.

In einem alternativen nicht erfindungsgemäßen Verfahren hingegen wird die Biomasse in der anaeroben biologischen Reinigungsstufe festgesetzt, das heißt immobilisiert. Dazu eignen sich beispielsweise verzweigte Strukturen mit großer und rauer Oberfläche, worauf sich die Biomasse festsetzen kann. Auf geeignete Schlammrückhaltesysteme kann dann gegebenenfalls verzichtet werden. Zweckmäßigerweise wird bei immobilisierter Biomasse die anaerobe biologische Reinigung einstufig, beispielsweise in einem einzigen Reaktorgefäß, durchgeführt. Die Kompartimentierung in zwei- beziehungsweise mehrstufige Reaktorsysteme erübrigt sich dann.

In bevorzugter Ausführung enthält das erfindungsgemäße Verfahren mindestens einen weiteren Schritt, worin der in dem Abwasser enthaltende Phosphor, der beispielsweise als anorganisches Phosphat vorliegt, ganz oder teilweise entfernt, das heißt eliminiert wird. Der bevorzugte weitere Schritt der Phosphor-Eliminierung findet bevorzugt im Anschluss an das Durchlaufen der erfindungsgemäßen anaeroben biologischen Reinigungsstufe statt. Die Eliminierung des Phosphors kann in an sich bekannter Weise durchgeführt werden und der Fachmann wird, je nach Anwendungsgebiet und Zweckmäßigkeit, das geeignete Verfahren wählen. Vorzugsweise findet die Fällung von Phosphat als schwerlösliche Aluminium-, Magnesium- und/oder Eisenverbindung statt.

Bevorzugt wird die Eliminierung des Phosphors durchgeführt mittels Fällung als Magnesiumammoniumphosphat (MAP). Dies geschieht beispielsweise durch Zudosierung entsprechender Magnesiumsalze, wodurch das Phosphat mit ebenfalls im Abwasser enthaltenden Ammoniumionen als schwerlösliches Doppelsalz ausfällt. Dieses kann dann mit geeigneten Maßnahmen aus dem Abwasser entfernt werden. Alternativ oder zusätzlich kann Phosphat durch Zugabe einer Eisen(III)chloridlösung als Eisen(III)phosphat gefällt werden. Die Entfernung des schwerlöslichen Eisenphosphats erfolgt in an sich bekannter Weise. Vorzugsweise ist die erfindungsgemäße Vorrichtung weiter mit einer Vorrichtung zur Elimination von Phosphor ausgestaltet, wobei die Vorrichtung einen Reaktor sowie mindestens eine Einrichtung zur Dosierung von Salzlösung ausgewählt aus Eisen(III)salz und Magnesiumsalz zur Fällung anorganischer Phosphorverbindungen aufweist.

In bevorzugter Ausführung wird der im Abwasser enthaltende Stickstoff in einem weiteren Schritt aus dem Abwasser teilweise oder vollständig entfernt, das heißt eliminiert. Dazu sind alle dem Fachmann bekannte Verfahren zur Stickstoff-Eliminierung einsetzbar. Der Fachmann wird, je nach Anwendungsgebiet und Zweckmäßigkeit, das geeignete Verfahren zur Stickstoff-Eliminierung wählen. So kann die Eliminierung des Stickstoffs in einer nach-/oder vorgeschalteten aeroben biologischen Reinigungsstufe mittels aerober Nitrifikation/Denitrifikation in an sich bekannter Weise stattfinden.

In einer bevorzugten Variante wird zusätzlich oder alternativ der Stickstoff eliminiert, indem Ammonium-Stickstoff an einem Zeolithen, vorzugsweise Klinoptilolith, ausgetauscht wird. Vorzugsweise wird der Zeolith vom Abwasser im Hauptstrom durchströmt. Dabei ist vorgesehen, dass der mit Ammonium beladene Zeolith in einem weiteren Schritt mit Regenerationslösung, beispielsweise Natriumchloridlösung, regeneriert wird, wodurch eine Ammonium-haltige Regenerationslösung erhalten wird. In einer Variante wird die Regeneration bei erhöhten Temperaturen durchgeführt. Die Ammonium-haltige Regenerationslösung wird in einem weiteren Schritt, vorzugsweise durch Luftstrippung, vom Ammonium befreit, der als Ammoniak aus der Ammonium-haltigen Regenerationslösung austritt. Die Luftstrippung des Ammoniaks findet in an sich bekannter Weise statt. Dabei findet die Strippung des Ammoniaks im Nebenstrom aus der Regenerationslösung statt, worin der Ammonium-Stickstoff in hoher Konzentration vorliegt. Vorteilhafterweise ist die Strippung dann besonders effektiv. Es ist bevorzugt vorgesehen, dass die gestrippte Regenerationslösung teilsweise oder vollständig erneut zur Regeneration des Leoliths verwendet wird. Zur eigentlichen Rückgewinnung des Ammonium-stickstoffs nach der Luftstrippung wird vorzugsweise ein saurer Wäscher eingesetzt.

Vorzugsweise wird der Schritt der Stickstoff-Eliminierung nach dem oder im Anschluss an den Schritt der Phosphor-Eliminierung durchgeführt. Auch die umgekehrte Reihenfolge ist vorgesehen; der Fachmann wird die Reihenfolge der Schritte je nach Anwendungsgebiet und Zweckmäßigkeit wählen.

Vorzugsweise ist die zum erfindungsgemäßen Verfahren verwendete Vorrichtung weiter mit einer Vorrichtung zur Elimination von Stickstoff ausgestattet, wobei die Vorrichtung mindestens eine Zeolith-Kolonne, eine Regenerationseinheit und eine Vorrichtung zur Ammoniak-Luftstrippung aus der Regenerationslösung aufweist.

Ein weiterer Gegenstand der Erfindung ist auch ein Verfahren zur Gewinnung von Biogas aus Abwasser, wobei die erfindungsgemäße anaerobe biologische Reinigung mittels psychrophiler Mikroorganismen eingesetzt wird, um gleichzeitig zur Reinigung des Abwassers bei der Methanogenese entstehendes Biogas zu gewinnen. Das Verfahren enthält zumindest die Schritte Bereitstellen von Abwasser, Durchführen der anaeroben biologischen Abwasserreinigung mittels psychrophiler Biomasse nach dem erfindungsgemäßen Verfahren und Aufsammeln beziehungsweise Auffangen der bei der anaeroben biologischen Reinigung in der Biomasse entstehenden Biogase, vor allern Methan und Kohlenstoffdioxid. Vorzugsweise ist im Biogas ca. 60 % Methan und ca. 40 % Kohlenstoffdioxid enthalten. Das gesammelte Biogas wird bevorzugt als Brennstoff, beispielsweise in Blockheizkraftwerken, zur Stromerzeugung und zur Heizung eingesetzt. Gegenstand der vorliegenden Erfindung ist auch die Verwendung einer Vorrichtung, die zur anaeroben biologischen Reinigung von Abwasser geeignet ist und/oder für die anaerobe biologische Reinigung von Abwasser verwendet wird. Für die anaerobe biologische Reinigung von Abwasser, vorzugsweise mittels einstufiger Methanisierung, geeignet und/oder verwendet wird erfindungsgemäß eine Vorrichtung, die zumindest folgende Elemente enthält oder vorzugsweise daraus besteht:
(a) Mindestens einen Reaktor, wobei der Reaktor zumindest mindestens einen ersten Zulauf für das zu reinigende Abwasser, vorzugsweise gegebenenfalls mindestens einen zweiten Zulauf für den Rücklauf von Schlamm, gegebenenfalls mindestens einen oberen Auslass für Biogas und mindestens einen Ablauf aufweist oder daraus besteht, sowie
(b) mindestens eine Mikrofiltrationsvorrichtung, vorzugsweise einen Rotationsscheibenfilter, zur Aufkonzentrierung des bei der biologischen Reinigung anfallenden Schlamms, wobei die Mikrofiltrationsvorrichtung zumindest mindestens einen Feedzulauf, mindestens einen Filtratablauf für das gereinigte Abwasser, mindestens eine wellennahe Gasabführung und mindestens einen Retentatablauf für den aufkonzentrierten Schlamm aufweist oder daraus besteht.

Bevorzugt ist die Mikrofiltrationsvorrichtung zusätzlich mit mindestens einem Gasauslass versehen, um das im abgetrennten Schlamm enthaltene Gas, im Wesentlichen Biogas, abzuführen. Das abgeführte Gas wird vorzugsweise mit der Biogasabführung des Reaktors zusammengeführt. Vorzugsweise ist die Mikrofiltrationsvorrichtung als Rotationsscheibenfilter ausgeführt. Die Ableitung des sich in der Mikrofiltrationsvorrichtung ansammelnden Gases wird dann im Wesentlichen durch rotationsachsennahe Durchbrechungen ermöglicht. Dabei wird im Betrieb des Rotationsscheibenfilters das sich bei der Rotation in Achsennähe ansammelnde Gas durch die Durchbrechungen entlang der Rotationsachse über eine achsennahe Auslassöffnung drainiert. Bevorzugt weist die erfindungsgemäß vorgesehene Mikrofiltrationsvorrichtung zusätzlich mindestens einen Auslass für sich ansammelndes Biogas auf.

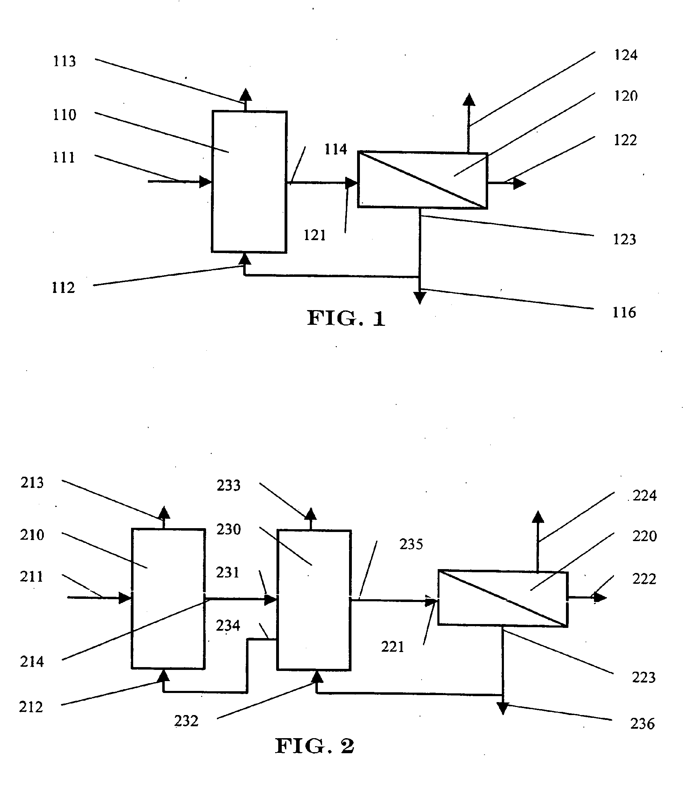

Bevorzugt ist die im deutschen Patent DE 101 54 549 besonders Figuren 1, 2 und 3 und die zugehörige Figurenbeschreibung beschriebene Filtervorrichtung vorgesehen. Diese Filtervorrichtung ist ein Rotationsscheibenfilter, der über eine zusätzliche Gasabführung verfügt.

Erfindungsgemäß ist der Ablauf des Reaktors mit dem Feedzulauf der Mikrofiltrationsvorrichtung derart verbunden, dass Schlamm das heißt die Biomasse, der aus dem Reaktor austritt, in die Mikrofiltrationsvorrichtung geführt wird. Erfindungsgemäß ist der Retentatablauf der Mikrofiltrationsvorrichtung derart mit dem zweiten Zulauf des Reaktors verbunden, dass der zurückgehaltene beziehungsweise aufkonzentrierte Schlamm in den Reaktor zurückgeführt werden kann. Bevorzugt wird der zurückgehaltene Schlamm direkt in den Reaktor, vorzugsweise über einen (separaten) zweiten Zulauf zurückgeführt. In einer Variante wird der Schlamm unmittelbar in den Feedzulauf der erfindungsgemäßen biologischen Reinigungsstufe zurückgeführt.

Vorzugsweise ist an Reaktor und/oder Mikrofiltrationsvorrichtung mindestens ein Auslass für überschüssigen Schlamm vorgesehen; bevorzugt ist der Schlammauslass an der Schlammrückführung von der Mikrofiltrationsvorrichtung in den Reaktor angeordnet.

Ein Aufbau mit Bioreaktor und darin integrierter Mikrofiltrationsvorrichtung eignet sich bevorzugt in Fällen nicht allzu hoher Belastung des zu reinigenden Abwassers mit organischen Bestandteilen, beispielsweise bis etwa 1000 mg/l CSB, als einstufige vollständige, das heißt notwendige und ausreichende, Einheit für die biologische Reinigungsstufe einer Abwasserreinigungsanlage. In einer bevorzugten Variante ist die Mikrofiltrationseinheit im Volumen des Bioreaktors installiert.

Ein weiterer Gegenstand der Erfindung ist die Verwendung einer Vorrichtung, die sich von der vorbeschriebenen Vorrichtung ableitet, vor allem für die Reinigung von Abwasser mittels zweistufiger Methanisierung geeignet ist und/oder eingesetzt wird. Diese für das erfindungsgemäße Verfahren geeignete Vorrichtung enthält zumindest folgende Bestandteile oder besteht vorzugsweise daraus:
(a) Mindestens einen ersten Reaktor, wobei der erste Reaktor zumindest mindestens einen (ersten) Zulauf für das zu reinigende Abwasser, gegebenenfalls mindestens einen (zweiten) Zulauf für Schlamm, gegebenenfalls mindestens einen oberen Auslass für Biogas und mindestens einen Ablauf aufweist oder daraus besteht,
(b) mindestens einen zweiten Reaktor, wobei der zweite Reaktor zumindest mindestens einen ersten Zulauf, gegebenenfalls mindestens einen zweiten Zulauf für Schlamm, gegebenenfalls mindestens einen oberen Auslass für Biogas und mindestens einen Ablauf aufweist oder daraus besteht, und
(c) mindestens eine Mikrofiltrationsvorrichtung, vorzugsweise einen Rotationsscheibenfilter, zur Aufkonzentrierung des bei der biologischen Reinigung anfallenden Schlamms, wobei die Mikrofiltrationsvorrichtung zumindest mindestens einen Feedzulauf, mindestens einen Filtratablauf für das gereinigte Abwasser, mindestens einen Retentatablauf für den aufkonzentrierten Schlamm und vorzugsweise mindestens einen Auslass für Biogas aufweist oder daraus besteht.

Der Ablauf des ersten Reaktors dieser Vorrichtung steht bevorzugt mit dem ersten Zulauf des zweiten Reaktors in Verbindung, sodass Schlamm aus dem ersten Reaktor in den zweiten Reaktor geführt werden kann.

Mindestens ein Ablauf des zweiten Reaktors steht bevorzugt mit dem ersten und/oder einem separaten zweiten Zulauf des ersten Reaktors in Verbindung, sodass Schlamm aus dem zweiten Reaktor in den ersten Reaktor zurückgeführt werden kann. Alternativ oder zusätzlich steht bevorzugt mindestens ein Ablauf des zweiten Reaktors mit dem Feedzulauf der Mikrofiltrationsvorrichtung in Verbindung.

Der Retentatablauf der Mikrofiltrationsvorrichtung steht bevorzugt mit dem ersten oder einem separaten zweiten Zulauf des zweiten Reaktors in Verbindung, sodass aufkonzentrierter Schlamm in den zweiten Reaktor zurückgeführt werden kann. In einer bevorzugten Variante steht der Retentatablauf der Mikrofiltrationsvorrichtung zusätzlich oder bevorzugt alternativ mit dem ersten und/oder einem separaten zweiten Zulauf des ersten Reaktors in Verbindung, sodass aufkonzentrierter Schlamm in den ersten Reaktor zurückgeführt werden kann. In einer bevorzugten Ausführung dieser Variante steht der Retentatablauf mit einem zweiten Zulauf des ersten Reaktors in Verbindung.

Der bevorzugt in der Mikrofiltrationsvorrichtung vorgesehene Auslass für Biogas steht vorzugsweise mit den oberen Auslässen der Reaktoren in Verbindung, sodass Biogas gesammelt und abgeführt werden kann.

Vorzugsweise ist an Reaktor und/oder Mikrofiltrationsvorrichtung mindestens ein Auslass für überschüssigen Schlamm vorgesehen; bevorzugt ist der Schlammauslass an der Schlammrückführung von der Mikrofiltrationsvorrichtung in den Reaktor angeordnet.

Vorzugsweise enthalten die vorstehend beschriebenen Vorrichtungen weiter mindestens eine Vorrichtung zur Elimination von Phosphor, wobei die Vorrichtung einen Reaktor sowie mindestens eine Einrichtung zur Dosierung von Salzlösung ausgewählt aus Eisen(III)salz/Magnesiumsalz zur Fällung anorganischer Phosphorverbindungen aufweist.

Vorzugsweise enthalten die vorstehend beschriebenen Vorrichtungen weiter mindestens eine Vorrichtung zur Elimination von Stickstoff, wobei die Vorrichtung mindestens eine Zeolith-Kolonne, welche bevorzugt von dem zu reinigenden Abwasser im Hauptstrom durchflossen wird, eine Regenerationseinheit, vorzugsweise mit Vorlage für Regenerationslösung und Dosierungseinheit, zur Regeneration der mit Ammonium beladenen Austauschersäule und eine Vorrichtung zur Ammoniak-Luftstrippung aus der Regenerationslösung aufweist. Vorzugsweise ist in Verbindung mit der Luftstrippungseinheit Mindestens ein saurer Wäscher zur Lösung des gestrippten Ammoniaks vorgesehen.

### Ausführungsbeispiele

Die vorliegende Erfindung wird anhand der nachstehenden Beispiele und Figuren näher erläutert. Die Beispiele sind nicht beschränkend zu verstehen.
Figur 1 zeigt ein Schema einer im erfindungsgemäßen (Verfahren verwendete) Vorrichtung mit mindestens einem Reaktor (110) und mindestens einer Mikrofiltrationsvorrichtung (120). Der Reaktor (110) weist einen (ersten) Zulauf (111) für zu reinigendes Abwasser, einen (zweiten) Zulauf (112) für Schlamm, einen (oberen) Auslass (113) für Biogas und einen Ablauf (114) auf. Die Mikrofiltrationsvorrichtung (120) weist einen Feedzulauf (121), einen Filtratablauf (122), einen Retentatablauf (123) und einen Auslass (124) für Biogas auf. Der Ablauf (114) des Reaktors (110) ist mit dem Feedzulauf (121) verbunden, sodass Schlamm aus dem Reaktor (110) in die Mikrofiltrationsvorrichtung (120) eintreten kann. Der Retentatablauf (123) ist mit dem (zweiten) Zulauf (112) des Reaktors (110) verbunden, sodass aufkonzentrierter Schlamm in den Reaktor (110) zurückgeführt werden kann. Ein weiterer Auslass (116) ist für den Austrag von überschüssigem Schlamm vorgesehen.
Figur 2 zeigt eine weitere Vorrichtung, die einen ersten Reaktor (210), einen zweiten Reaktor (230) und mindestens eine Mikrofiltrationsvorrichtung (220) aufweist. Der erste Reaktor (210) weist einen ersten Zulauf (211) für zu reinigendes Abwasser, einen zweiten Zulauf (212) für Schlamm, einen oberen Auslass (213) für Biogas und einen Ablauf (214) auf. Der zweite Reaktor (230) weist einen ersten Zulauf (231), einen zweiten Zulauf (232) für Schlamm, einen oberen Auslass (233) für Biogas, einen ersten Ablauf (234) und einen zweiten Ablauf (235) auf. Die Mikrofiltrationsvorrichtung (220) weist einen Feedzulauf (221), einen Filtratablauf (222) für das gereinigte Abwasser, einen Retentatablauf (223) für den aufkonzentrierten Schlamm und einen Auslass (224) für Biogas auf. Der erste Ablauf (234) des zweiten Reaktors (230) ist mit dem zweiten Zulauf (212) des ersten Reaktors (210) verbunden, sodass Schlamm aus dem zweiten Reaktor in den ersten Reaktor zurückgeführt werden kann. Der zweite Ablauf (235) des zweiten Reaktors (230) ist mit dem Feedzulauf (221) der Mikrofiltrationsvorrichtung (220) verbunden, sodass Schlamm aus dem zweiten Reaktor (230) in die Mikrofiltrationsvorrichtung (220) eintreten kann. Der Retentatablauf (223) der Mikrofiltrationsvorrichtung (220) ist mit dem zweiten Zulauf (232) des zweiten Reaktors (230) verbunden, sodass aufkonzentrierter Schlamm in den zweiten Reaktor zurückgeführt werden kann. Ein weiterer Auslass (236) ist für den Austrag von überschüssigem Schlamm vorgesehen.

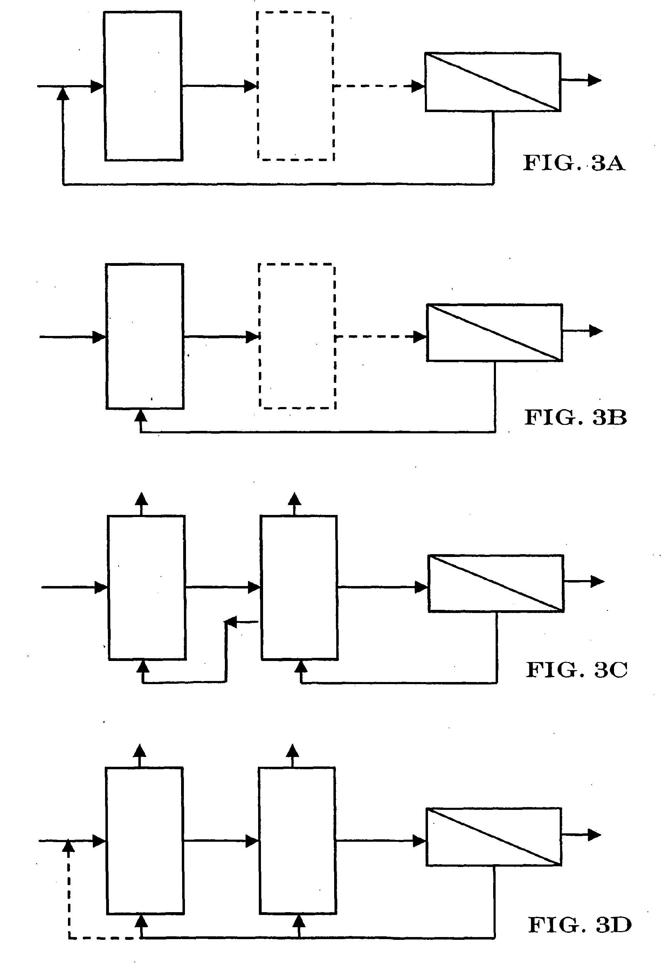

Figur 3 zeigt eine schematische Darstellung alternativer Ausführungsformen der verwendeten Vorrichtungen, insbesondere nach den Figuren 1 oder 2. Dabei wird in einer Variante die abgetrennte Schlammfraktion an einem ein- oder mehrstufigen System in den Feedzulauf der ersten Stufe (Figur 3A) und/oder in den separaten Zulauf der ersten Stufe (Figur 3B) zurückgeführt. Figur 3C zeigt schematisch die in der Anordnung nach Figur 2 bevorzugt verwirklichte Schlammrückführung. Wie in Figur 3D schematisch dargestellt, erfolgt in einer weiteren bevorzugten Variante die Schlammrückführung in alle Reaktoren; eine zusätzliche Schlammrückführung aus dem nachgeschalteten Reaktor in den vorgeschalteten beziehungsweise ersten Reaktor ist dann nicht erforderlich.

### Beispiel 1: Versuchsanlage zur anaeroben Reinigung von Abwasser im Technikumsmaßstab

Es wurde eine Versuchsanlage zur anaeroben Reinigung von Abwasser mittels psychrophiler Biomasse im Technikumsmaßstab aufgebaut. Als Testabwasser wurde entweder kommunales Abwasser mit hoher organischer Belastung, das beispielsweise durch Mischen mit zerkleinerten Küchenabfällen erzeugt werden kann, oder stark konzentriertes synthetisches Abwasser, jeweils mit einem CSB-Wert von etwa 3500 bis 5000 mg/l bereitgestellt.

Es wurde die in Figur 2 näher beschriebene Anlage aus zwei hintereinander geschalteten Bioreaktoren (210; 230) sowie einer nachgeschalteten Mikrofiltrationsvorrichtung (220) in Form eines Rotationsscheibenfilters zum Rückhalt der aus den Bioreaktoren entweichenden Biomasse aufgebaut. Die Anlage fasst insgesamt 21 l. Die Bewegung der Flüssigkeiten in den einzelnen Komponenten der Vorrichtung wird teilweise mittels hydrostatischem Druckgefälle teilweise mittels eingebauter Pumpen bewerkstelligt. Das in den Reaktoren und der Mikrofiltrationsvorrichtung vorhandene Biogas wird gesammelt und abgeführt.

Ergebnisse: Mittels der nachgeschalteten Rotationsscheibenfilter konnte sich die Biomasse in den Bioreaktoren auf weit über 20 g/l aufkonzentrieren lassen. Wurde der Anlage stark konzentriertes organisch belastetes Abwasser (CSB-Wert 3500 bis 5000 mg/l) zugeführt, konnte ein CSB-Abbaugrad von deutlich über 95 % erreicht werden. Dabei gelang es, die CSB-Werte im Ablauf der Anlage unter 100 mg/l, teilweise unter 75 mg/l zu halten. Bei einer Wassertemperatur im Zulauf von etwa 20°C und bei einer Verweilzeit von etwa 29 Stunden im Gesamtsystem betrug die Raumbelastung etwa 3 bis 4 g/l pro Tag CSB. Die Raumbelastung lag damit etwa 5-mal höher als bei bekannten aeroben Belebtschlammverfahren. Gleichzeitig konnte ein Ertrag an Biogas (60 % Methan und 40 % Kohlenstoffdioxid) von etwa 20 l pro Tag erreicht werden. Dies entspricht einer Ausbeute von etwa 0,35 l Gas pro 1 g abgebautem CSB.

### Beispiel 2: Elimination von Stickstoff

Zusätzlich zu der in Figur 2 dargestellten und im Beispiel 1 betriebenen Vorrichtung wurde ein weiterer Reinigungsschritt realisiert, worin mittels MAP-Fällung Phosphat aus dem die erfindungsgemäße anaerobe biologische Reinigungsstufe durchlaufende Abwasser entfernt werden kann.

Zur Entfernung des Stickstoffs, der im Abwasser hauptsächlich in Form von Ammonium vorliegt, wird das die erfindungsgemäße anaerobe biologische Reinigungsstufe verlassende Abwasser im Hauptstrom durch einen Ammoniumaustauscher wie Zeolith, beispielsweise Klinoptilolith, geführt. Die Ammonium-Aufnahme in den Zeolith erfolgt bei Abwassertemperatur, beispielsweise Zimmertemperatur.

Der mit Ammonium beladenene Zeolith wird diskontinuierlich mit einer hochkonzentrierten Natriumchloridlösung in verschiedenen Ansätzen jeweils bei Normaltemperatur oder bei verschiedenen erhöhten Temperaturen regeneriert. Die Ammonium-haltige Natriumchloridlösung wird anschließend in einer Luftstrippungsanlage vom Ammonium befreit. Der bei der Luftstrippung entstehende Ammoniak wird in einem sauren Wäscher als Salz gebunden.

Ergebnisse: Es gelingt, den Ablauf der erfindungsgemäßen anaeroben biologischen Abwasserreinigung vollständig vom Ammonium zu befreien. Es werden beispielsweise Beladungen von etwa 4 bis 7 g Ammoniumstickstoff pro kg Zeolith erzielt. Eine Säule mit etwa 7 kg Zeolith kann dabei mit Volumenströmen von etwa 40 l pro Stunde beschickt werden.

Es gelingt weiter, die beladene Säule nahezu vollständig zu regenerieren. Dabei wird das Ammonium in der Regenerationslösung aufkonzentriert, so dass eine effektive Luftstrippung der Regenerationslösung durchgeführt werden kann. Durch die Luftstrippung wird die Regenerationslösung weitgehend NH₄ befreit und kann erneut zur Regeneration eingesetzt werden. Das NH₄ wird als Salz zurückgewonnen und kann so als Dünger eingesetzt werden.

## Patentansprüche

1. Verfahren zur Reinigung von Abwasser, enthaltend mindestens einen Schritt der anaeroben biologischen Reinigung, wobei die anaerobe biologische Reinigung im kontinuierlichen Betrieb in mindestens einem volldurchmischtem Bioreaktor mittels Biomasse erfolgt, wobei die Biomasse ausgewählt ist aus psychrophilen Mikroorganismen, wobei die Biomasse durch Mikrofiltration aufkonzentriert und in die anaerobe biologische Reinigung zurückgeführt wird und wobei die Biomasse im Bioreaktor eine Konzentration von 25 g/l oder mehr aufweist.

2. Verfahren nach Anspruch 1, wobei die psychrophilen Mikroorganismen ein Temperaturoptimum von unter 35 °C, bevorzugt unter 25°C, aufweisen.

3. Verfahren nach Anspruch 1 oder 2, wobei die anaerobe biologische Reinigung als einstufige Methanisierung durchgeführt wird.

4. Verfahren nach Anspruch 1 oder 2, wobei die anaerobe biologische Reinigung als zweistufige Methanisierung durchgeführt wird.

5. Verfahren nach einem der vorhergehenden Ansprüche, wobei die Biomasse eine Konzentration von 25 bis 100 g/l aufweist.

6. Verfahren nach einem der vorhergehenden Ansprüche, wobei die Biomasse in dem zu reinigenden Abwasser suspendiert ist.

7. Verfahren nach einem der vorhergehenden Ansprüche, wobei der Schlamm durch Mikrofiltration mittels Rotationsscheibenfilter aufkonzentriert wird.

8. Verfahren nach einem der vorhergehenden Ansprüche, enthaltend den weiteren Schritt der Phosphor-Eliminierung.

9. Verfahren nach Anspruch 8, wobei die Eliminierung des Phosphors ausgewählt ist aus Fällung als Magnesiumammoniumphosphat (MAP) und Fällung als Eisen(III)phosphat.

10. Verfahren nach einem der vorhergehenden Ansprüche, enthaltend den weiteren Schritt der Stickstoff-Eliminierung.

11. Verfahren nach Anspruch 10, wobei die Eliminierung des Stickstoffs durch Austausch des Ammonium-Stickstoffs am Zeolith erfolgt.

12. Verfahren nach Anspruch 11, wobei der mit Ammonium-Stickstoff beladene Zeolith in einem weiteren Schritt mit Regenerationslösung regeneriert wird und in einem weiteren Schritt der Ammonium-Stickstoff aus der Regenerationslösung durch Luftstrippung entfernt und Ammoniak erhalten wird.

13. Verfahren nach Anspruch 12, wobei die Regenerationslösung nach der Luftstrippung zumindest teilweise erneut zur Regeneration des Zeoliths verwendet wird.

14. Verfahren zur Gewinnung von Biogas aus Abwasser, enthaltend die Schritte:
Bereitstellen von Abwasser,
anaerobe biologische Abwasserreinigung mittels psychrophiler Biomasse nach dem Verfahren nach einem der Ansprüche 1 bis 13 und
Sammeln der bei der anaeroben biologischen Reinigung in der Biomasse entstehenden Biogase.

15. Verwendung einer Vorrichtung, geeignet zur anaeroben biologischen Reinigung von Abwasser mittels einstufiger Methanisierung in dem Verfahren nach Anspruch 1 bis 14, wobei die Vorrichtung enthält:
mindestens einen Reaktor (110), wobei der Reaktor einen Zulauf (111) für zu reinigendes Abwasser, einen Zulauf (112) für Schlamm, einen oberen Auslass (113) für Biogas und einen Ablauf (114) aufweist, und
mindestens eine Mikrofiltrationsvorrichtung (120), wobei die Mikrofiltrationsvorrichtung einen Feedzulauf (121), einen Filtratablauf (122) und einen Retentatablauf (123) für den aufkonzentrierten Schlamm aufweist,
und wobei der Ablauf (114) mit dem Feedzulauf (121) verbunden ist, sodass Schlamm aus dem Bioreaktor in die Mikrofiltrationsvorrichtung geführt werden kann und der Retentatablauf (123) mit dem Zulauf (112) verbunden ist, sodass aufkonzentrierter Schlamm in den Bioreaktor zurückgeführt werden kann.

16. Verwendung einer Vorrichtung, geeignet zur anaeroben biologischen Reinigung von Abwasser mittels zweistufiger Methanisierung in dem Verfahren nach Anspruch 1 bis 14, wobei die Vorrichtung enthält:
mindestens einen ersten Reaktor (210), wobei der erste Reaktor einen ersten Zulauf (211) für zu reinigendes Abwasser, einen zweiten Zulauf (212) für Schlamm, einen oberen Auslass (213) für Biogas und einen Ablauf (214) aufweist,
mindestens einen zweiten Reaktor (230), wobei der zweite Reaktor einen ersten Zulauf (231), einen zweiten Zulauf (232) für Schlamm, einen oberen Auslass (233) für Biogas, einen ersten Ablauf (234) und gegebenenfalls einen zweiten Ablauf (235) aufweist, und
mindestens eine Mikrofiltrationsvorrichtung (220), wobei die Mikrofiltrationsvorrichtung einen Feedzulauf (221), einen Filtratablauf (222) für das gereinigte Abwasser und einen Retentatablauf (223) für den aufkonzentrierten Schlamm aufweist.

17. Verwendung nach Anspruch 16, wobei in der Vorrichtung der erste Ablauf (234) des zweiten Reaktors (230) mit dem zweiten Zulauf (212) des ersten Reaktors (210) verbunden ist, sodass Schlamm aus dem zweiten Reaktor in den ersten Reaktor zurückgeführt werden kann.

18. Verwendung nach Anspruch 16 oder 17, wobei in der Vorrichtung der erste Ablauf (234) oder der zweite Ablauf (235) des zweiten Reaktors (230) mit dem Feedzulauf (221) der Mikrofiltrationsvorrichtung (220) verbunden ist.

19. Verwendung nach einem Ansprüche 16 bis 18, wobei in der Vorrichtung der Retentatablauf (223) der Mikrofiltrationsvorrichtung (220) mit dem zweiten Zulauf (232) des zweiten Reaktors (230) verbunden ist, sodass aufkonzentrierter Schlamm in den zweiten Reaktor zurückgeführt werden kann.

20. Verwendung nach einem Ansprüche 16 bis 19, wobei in der Vorrichtung der Retentatablauf (223) der Mikrofiltrationsvorrichtung (220) mit dem zweiten Zulauf (212) des ersten Reaktors (210) verbunden ist, sodass aufkonzentrierter Schlamm in den ersten Reaktor zurückgeführt werden kann.

## Claims

1. Method for the purification of wastewater containing at least a step of anaerobic biological purification, wherein the anaerobic biological purification is effected in continuous operation in at least one complete-mix bioreactor by means of biomass, wherein the biomass is selected from psychrophilic micro-organisms, the biomass being concentrated by means of microfiltration and being returned into the anaerobic biological purification, and wherein the biomass in the bioreactor has a concentration of 25 g/I or more.

2. Method according to claim 1, wherein the psychrophilic micro-organisms have a temperature optimum of less than 35 °C, preferably less than 25 °C.

3. Method according to claim 1 or 2, wherein the anaerobic biological purification is carried out as a single-stage methanisation.

4. Method according to claim 1 or 2, wherein the anaerobic biological purification is carried out as a two-stage methanisation.

5. Method according to one of the preceding claims, wherein the biomass has a concentration of 25 to 100 g/l.

6. Method according to one of the preceding claims, wherein the biomass is suspended in the wastewater to be purified.

7. Method according to one of the preceding claims, wherein the sludge is concentrated by microfiltration by means of rotating disk filters.

8. Method according to one of the preceding claims, comprising the further step of phosphorus elimination.

9. Method according to claim 8, wherein the elimination of phosphorus is selected from precipitation as ammonium magnesium phosphate (AMP) and precipitation as iron(III)phosphate.

10. Method according to one of the preceding claims, comprising the further step of nitrogen elimination.

11. Method according to claim 10, wherein the nitrogen elimination is effected by exchanging the nitrogen of the ammonium on the zeolite.

12. Method according to claim 11, wherein, in a further step, the zeolite loaded with ammonium nitrogen is regenerated with regenerating solution and, in a further step, the ammonium nitrogen is removed from the regenerating solution by air stripping, and ammonia is obtained.

13. Method according to claim 12, wherein after the air stripping the regenerating solution is at least in part re-used for the regeneration of the zeolite.

14. Method for obtaining biogas from wastewater, comprising the steps of:
providing wastewater,
anaerobic biological purification of the wastewater by means of psychrophilic biomass with the method according to one of claims 1 to 13 and
collecting the biogases generated in the biomass during the anaerobic biological purification.

15. Use of an apparatus suitable for anaerobic biological purification of wastewater by means of single-stage methanisation in the method according to claim 1 to 14, the apparatus comprising:
at least one reactor (110), the reactor having an inlet (111) for the wastewater to be purified, an inlet (112) for sludge, an upper outlet (113) for biogas and a drain (114), and
at least one microfiltration apparatus (120), the microfiltration apparatus having a feed inlet (121), a filtrate drain (122) and a retentate drain (123) for the concentrated sludge,
and wherein the drain (114) is connected to the feed inlet (121) so that sludge from the bioreactor can be fed into the microfiltration apparatus, and the retentate drain (123) is connected to the inlet (112) so that concentrated sludge can be returned into the bioreactor.

16. Use of an apparatus suitable for anaerobic biological purification of wastewater by means of two-stage methanisation in the method according to claim 1 to 14, the apparatus comprising:
at least a first reactor (210), the first reactor having a first inlet (211) for wastewater to be purified, a second inlet (212) for sludge, an upper outlet (213) for biogas and a drain (214),
at least a second reactor (230), the second reactor having a first inlet (231), a second inlet (232) for sludge, an upper outlet (233) for biogas, a first drain (234) and possibly a second drain (235), and
at least one microfiltration apparatus (220), the microfiltration apparatus having a feed inlet (221), a filtrate drain (222) for the purified wastewater and a retentate drain (223) for the concentrated sludge.

17. Use according to claim 16, wherein, in the apparatus, the first drain (234) of the second reactor (230) is connected to the second inlet (212) of the first reactor (210), so that sludge from the second reactor can be returned into the first reactor.

18. Use according to claim 16 or 17, wherein, in the apparatus, the first drain (234) or the second drain (235) of the second reactor (230) is connected to the feed inlet (221) of the microfiltration apparatus (220).

19. Use according to one of claims 16 to 18, wherein, in the apparatus, the retentate drain (223) of the microfiltration apparatus (220) is connected to the second inlet (232) of the second reactor (230) so that concentrated sludge can be returned into the second reactor.

20. Use according to one of claims 16 to 19, wherein, in the apparatus, the retentate drain (223) of the microfiltration apparatus (220) is connected to the second inlet (212) of the first reactor (210) so that concentrated sludge can be returned into the first reactor.

## Revendications

1. Procédé d'épuration des eaux usées, comportant au moins une étape de traitement biologique anaérobie, dans lequel le traitement biologique anaérobie s'effectue en exploitation continue dans au moins un bioréacteur sous agitation continue par biomasse, la biomasse étant sélectionnée parmi des micro-organismes psychrophiles, la biomasse étant concentrée par microfiltration et réintégrée dans le traitement biologique anaérobie, et la biomasse présentant une concentration de 25 g/l ou plus dans le bioréacteur.

2. Procédé selon la revendication 1, dans lequel les micro-organismes psychrophiles présentent une température optimale inférieure à 35 °C, de préférence inférieure à 25 °C.

3. Procédé selon la revendication 1 ou 2, dans lequel le traitement biologique anaérobie est réalisé par méthanisation à une étape.

4. Procédé selon la revendication 1 ou 2, dans lequel le traitement biologique anaérobie est réalisé par méthanisation à deux étapes.

5. Procédé selon l'une des revendications précédentes, dans lequel la biomasse présente une concentration de 25 à 100 g/l.

6. Procédé selon l'une des revendications précédentes, dans lequel la biomasse est en suspension dans les eaux usées à épurer.

7. Procédé selon l'une des revendications précédentes, dans lequel la boue est concentrée par microfiltration au moyen de filtres à disque rotatifs.

8. Procédé selon l'une des revendications précédentes, comportant l'étape supplémentaire d'élimination du phosphore.

9. Procédé selon la revendication 8, dans lequel l'élimination du phosphore est sélectionnée parmi la précipitation sous forme de phosphate d'ammonium de magnésium (MAP) et la précipitation sous forme de phosphate de fer (III).

10. Procédé selon l'une des revendications précédentes, comportant l'étape supplémentaire d'élimination de l'azote.

11. Procédé selon la revendication 10, dans lequel l'élimination de l'azote s'effectue par échange de l'azote d'ammonium sur la zéolithe.

12. Procédé selon la revendication 11, dans lequel la zéolithe chargée d'azote d'ammonium est régénérée dans une étape supplémentaire avec une solution de régénération, et l'azote d'ammonium est éliminé de la solution de régénération par stripage à l'air dans une étape supplémentaire, et de l'ammoniac est obtenu.

13. Procédé selon la revendication 12, dans lequel la solution de régénération est réutilisée au moins en partie après le stripage à l'air pour la régénération de la zéolithe.

14. Procédé d'obtention de biogaz à partir d'eaux usées, comportant les étapes de :
mise à disposition des eaux usées,
traitement biologique anaérobie des eaux usées par biomasse psychrophile selon le procédé d'une des revendications 1 à 13, et
collecte des biogaz formés dans la biomasse par le traitement biologique anaérobie.

15. Utilisation d'un dispositif adapté au traitement biologique anaérobie des eaux usées par méthanisation à une étape dans le procédé selon les revendications 1 à 14, dans laquelle le dispositif comprend :
au moins un réacteur (110), le réacteur présentant une amenée (111) pour les eaux usées à épurer, une amenée (112) pour la boue, une sortie supérieure (113) pour le biogaz et un conduit d'évacuation (114), et
au moins un dispositif de microfiltration (120), le dispositif de microfiltration présentant un conduit d'alimentation (121), un conduit d'évacuation de filtrat (122) et un conduit d'évacuation de rétentat (123) pour la boue concentrée,
et le conduit d'évacuation (114) étant relié au conduit d'alimentation (121), de sorte que la boue provenant du bioréacteur puisse être amenée dans le dispositif de microfiltration et le conduit d'évacuation de rétentat (123) étant relié à l'amenée (112), de sorte que la boue concentrée puisse être ramenée dans le bioréacteur.

16. Utilisation d'un dispositif adapté au traitement biologique anaérobie des eaux usées par méthanisation à deux étapes unique dans le procédé selon les revendications 1 à 14, dans laquelle le dispositif comprend :
au moins un premier réacteur (210), le premier réacteur présentant une première amenée (211) pour les eaux usées à épurer, une deuxième amenée (212) pour la boue, une sortie supérieure (213) pour le biogaz et un conduit d'évacuation (214),
au moins un deuxième réacteur (230), le deuxième réacteur présentant une première amenée (231), une deuxième amenée (232) pour la boue, une sortie supérieure (233) pour le biogaz, un premier conduit d'évacuation (234) et, le cas échéant, un deuxième conduit d'évacuation (235), et
au moins un dispositif de microfiltration (220), le dispositif de microfiltration présentant un conduit d'alimentation (221), un conduit d'évacuation de filtrat (222) pour les eaux usées épurées et un conduit d'évacuation de rétentat (223) pour la boue concentrée.

17. Dispositif selon la revendication 16, dans lequel, dans le dispositif, le premier conduit d'évacuation (234) du deuxième réacteur (230) est relié à la deuxième amenée (212) du premier réacteur (210), de sorte que la boue provenant du deuxième réacteur puisse être ramenée dans le premier réacteur.

18. Dispositif selon la revendication 16 ou 17, dans lequel, dans le dispositif, le premier conduit d'évacuation (234) ou le deuxième conduit d'évacuation (235) du deuxième réacteur (230) est relié au conduit d'alimentation (221) du dispositif de microfiltration (220).

19. Dispositif selon l'une des revendications 16 à 18, dans lequel, dans le dispositif, le conduit d'évacuation de rétentat (223) du dispositif de microfiltration (220) est relié à la deuxième amenée (232) du deuxième réacteur (230), de sorte que la boue concentrée puisse être ramenée dans le deuxième réacteur.

20. Dispositif selon l'une des revendications 16 à 19, dans lequel, dans le dispositif, le conduit d'évacuation de rétentat (223) du dispositif de microfiltration (220) est relié à la deuxième amenée (212) du premier réacteur (210), de sorte que la boue concentrée puisse être ramenée dans le premier réacteur.
